# EUROPEAN PATENT SPECIFICATION

(11) **EP 4 079 349 B1**
(45) Date of publication and mention of the grant of the patent: **18.03.2026**
(21) Application number: 20906457.5
(22) Date of filing: 22.12.2020
(51) Int. Cl.: A61M 5/31

(54) **SYRINGE-BARREL GRIP, BARREL ASSEMBLY, AND SYRINGE**
SPRITZENZYLINDERGRIFF, ZYLINDEREINHEIT UND SPRITZE
POIGNÉE DE CYLINDRE DE SERINGUE, ENSEMBLE CYLINDRE ET SERINGUE

(30) Priority: 27.12.2019 JP 2019239094
(43) Date of publication of application: 26.10.2022
(73) Proprietor: TERUMO Kabushiki Kaisha, Tokyo 151-0072 (JP)
(72) Inventor: FUJIWARA, Sho, Ashigarakami-gun, Kanagawa 259-0151 (JP); TAKEMOTO, Masafumi, Fujinomiya-shi, Shizuoka 418-0004 (JP)
(74) Representative: Casalonga
(86) International application number: PCT/JP2020/047803
(87) International publication number: WO 2021/132202

(56) References cited:
- EP-A1- 2 934 635
- EP-B1- 2 934 635
- FR-A1- 2 269 971
- JP-A- 2013 070 788
- JP-A- 2013 070 788
- JP-A- 2015 536 768
- JP-A- 2015 536 768
- US-A- 3 921 633
- US-A- 5 338 309
- US-A- 5 338 309
- US-A- 5 554 133
- US-A- 5 554 133

## Description

### BACKGROUND

### Technical Field

The present invention relates to a grip for a syringe barrel to be attached to a syringe barrel, a barrel assembly including such a grip for a syringe barrel, and a syringe.

### Related Art

Some drug-filled syringes (prefilled syringes) use a plunger that is not coupled to a gasket to prevent medical malpractice. In such a prefilled syringe, a clip (backstop) for preventing the plunger from moving to a proximal end side and falling off from the barrel is attached (Patent Document 1).

In addition, in order to facilitate gripping and operation of the syringe, it is also known to attach a clip (grip), having a finger hook portion protruding outward, to the proximal end portion of the barrel (Patent Document 2).

These clips are fitted to an outer peripheral surface of the barrel at a fitting portion having a tubular shape and firmly attached to the syringe so as not to rattle or come off during operation of the syringe.

### PRIOR ART DOCUMENTS

### PATENT DOCUMENTS

Patent Document 1: JP2015-517860 A (US2015-105734A1)
Patent Document 2: WO2017-073658 A (US2018-243509A1)

US 5 554 133 A and US 5 338 309 A disclose grips for syringe barrels with flange engagement and airflow slots.

### SUMMARY

### PROBLEMS TO BE SOLVED BY THE INVENTION

In recent years, for an ophthalmic application or the like, higher sterility of not only a drug to be administered and a part in a prefilled syringe in contact with the drug, but also whole of the prefilled syringe including a grip is required.

Therefore, the inventors have studied application of a surface sterilization method with a small heat load, such as the hydrogen peroxide sterilization, the ethylene oxide gas (EOG) sterilization, and the NO₂ sterilization, to a prefilled syringe to which a grip is attached. These surface sterilization methods are sterilization with gas (sterilization gas), and in order to achieve higher sterility, it is preferable that the sterilization gas is brought into contact with a wider area of the surface of the prefilled syringe.

Therefore, the present invention provides a grip for a syringe barrel capable of achieving higher sterility in the state of being attached to a syringe barrel, a barrel assembly and a syringe including such a grip for a syringe barrel.

### MEANS FOR SOLVING THE PROBLEMS

The invention is defined by the subject-matter of claim 1. Further embodiments are defined in the dependent claims.

In addition, the above object is achieved also by the following.

A barrel assembly including: a syringe barrel including a tubular body and a flange provided at a proximal end of the tubular body and projecting outward; and the above grip for a syringe barrel attached to the barrel.

In addition, the above object is achieved also by the following.

A syringe including: the above barrel assembly; a gasket slidably received in the barrel; and a plunger configured to move the gasket.

### BRIEF DESCRIPTION OF DRAWINGS

FIG. 1 is a front sectional view illustrating an example of a syringe of the present invention.
FIG. 2 is an enlarged sectional explanatory view of portion A in FIG. 1.
FIG. 3 is a perspective explanatory view of a grip for a syringe barrel used in the syringe in FIG. 1.
FIG. 4 is a right side view of the grip for a syringe barrel used in the syringe in FIG. 1.
FIG. 5 is a left side view of the grip for a syringe barrel used in the syringe in FIG. 1.
FIG. 6 is an enlarged cross-sectional explanatory view taken along line B-B in FIG. 1.
FIG. 7 is a perspective explanatory view illustrating a grip for a syringe barrel according to another example of the present invention.
FIG. 8 is an explanatory view of a syringe including a grip for a syringe barrel according to yet another example of the present invention, and is an enlarged cross-sectional explanatory view corresponding to FIG. 6.
FIG. 9 is a perspective explanatory view illustrating a grip for a syringe barrel according to yet another example of the present invention.
FIG. 10 is an explanatory view of a syringe including the grip for a syringe barrel in FIG. 9, and is an enlarged cross-sectional explanatory view corresponding to FIG. 6.
FIG. 11 is a perspective explanatory view illustrating a grip for a syringe barrel according to yet another example of the present invention.
FIG. 12 is an explanatory view of a syringe including the grip for a syringe barrel in FIG. 11, and is an enlarged cross-sectional explanatory view corresponding to FIG. 6.
FIG. 13 is a perspective explanatory view illustrating a grip for a syringe barrel according to yet another example of the present invention.
FIG. 14 is a perspective explanatory view illustrating a grip for a syringe barrel according to yet another example of the present invention.
FIG. 15 is a perspective explanatory view illustrating a grip for a syringe barrel according to yet another example of the present invention.
FIG. 16 is a perspective explanatory view illustrating a grip for a syringe barrel according to yet another example of the present invention.
FIG. 17 is an explanatory view of a syringe including the grip for a syringe barrel in FIG. 16, and is an enlarged cross-sectional explanatory view corresponding to FIG. 6.
FIG. 18 is an enlarged sectional explanatory view of portion C in FIG. 17.
FIG. 19 is an enlarged sectional view of a proximal end portion of the syringe according to yet another example of the present invention.
FIG. 20 is a perspective explanatory view of a grip for a syringe barrel used in the syringe in FIG. 19.
FIG. 21 is a perspective explanatory view illustrating a grip for a syringe barrel according to yet another example of the present invention.

### DESCRIPTION OF EMBODIMENTS

A barrel assembly including a grip for a syringe barrel and a syringe according to the present invention will be described with reference to examples illustrated in the drawings.

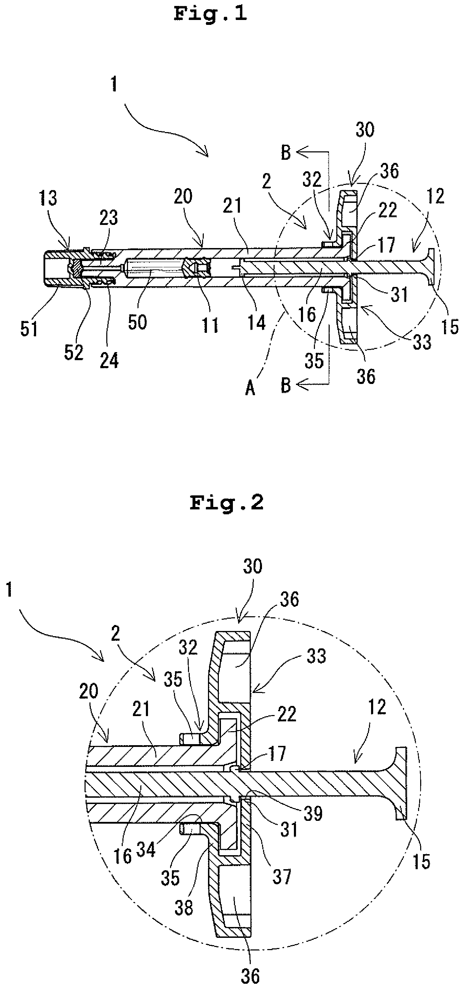

In the present example, the description will be given assuming that the right side in FIG. 1 (the side on which a flange 22 of a syringe barrel 20 is formed) is a proximal end side or an upper side, the left side in FIG. 1 (the side on which a nozzle portion 23 of the syringe barrel 20 is provided) is a distal end side or a lower side, and the left-right direction in FIG. 1 (the axial direction of the syringe barrel 20) is an axial direction (the axial direction of a tubular body 21 or a side wall portion 32 of a grip 30).

A syringe 1 of the present invention includes a barrel assembly 2 including the syringe barrel 20 (hereinafter also simply referred to as barrel 20) and the grip 30 for a syringe barrel (hereinafter also simply referred to as grip 30) attached to the barrel 20, a gasket 11 slidably received in the barrel 20, and a plunger 12 configured to move the gasket 11.

The barrel 20 includes the tubular body 21 and the flange 22 provided on the tubular body 21 and projecting outward (in a direction orthogonal to the axial direction of the barrel 20). A distal end opening portion (nozzle portion) 23 configured to discharge drug is provided at the distal end portion of the barrel 20. In addition, the barrel 20 includes a collar 24 that covers a proximal end side portion of the nozzle portion 23. A barrel-side screwing portion (female screwing portion) is formed on an inner surface of the collar 24.

The nozzle portion 23 is sealed by a seal cap 13, so that a drug 50 stored in the barrel 20 is prevented from leaking out and the drug 50 aseptically filled is prevented from coming into contact with the outside air.

Examples of constituent materials of the barrel 20 include various resins, e.g., polyolefin such as polyethylene and polypropylene, polystyrene, polyamide, polycarbonate, polyvinyl chloride, poly-(4-methylpentene-1), acrylic resin, acrylonitrile-butadiene-styrene copolymer, polyester such as polyethylene terephthalate, cyclic polyolefin polymer, and cyclic olefin copolymer. Among the above, resins such as polypropylene, cyclic polyolefin polymer, and cyclic olefin copolymer are preferable because these resins are easy to mold and have heat resistance. A cyclic olefin polymer or a cyclic olefin copolymer that has high transparency allowing the chemical solution filled inside to be visually confirmed from the outside and has heat resistance capable of withstanding high-pressure steam sterilization is particularly preferable as a material for forming the barrel 20.

The seal cap 13 includes a body part 51 having a rear end side hollow portion receiving the nozzle portion 23 of the barrel 20, and a seal member 52 received in the body part 51 and disposed at an upper end portion of the rear end side hollow portion. In addition, on an outer surface of the rear end side hollow portion, a cap side screw portion (male screw portion) capable of being screwed with a barrel side screw portion (female screw portion) formed on an inner surface of the collar 24 is formed.

Examples of molding materials of the seal cap 13 include various resins, e.g., polypropylene, polyethylene, polystyrene, polyamide, polycarbonate, polyvinyl chloride, poly-(4-methylpentene-1), acrylic resin, acrylonitrile-butadiene-styrene copolymer, polyester such as polyethylene terephthalate, and cyclic polyolefin. Among the above, resins such as polypropylene and cyclic polyolefin are preferable because these resins are easy to mold and have heat resistance.

As molding materials of the seal member 52, elastic materials, e.g., natural rubber, isoprene rubber, butyl rubber, butadiene rubber, fluororubber, synthetic rubber such as silicone rubber, and thermoplastic elastomer such as olefinic elastomer and styrene elastomer are preferable.

The gasket 11 received in the barrel 20 is made of elastic rubber or synthetic resin. The gasket 11 includes a body having a substantially cylindrical shape extending with substantially the same outer diameter as that of the gasket 11 and a plurality of annular ribs provided on an outer surface of the body. An outer surface of the annular rib is able to be in liquid-tight contact with an inner surface of the barrel 20 and is slidable in a liquid-tight state.

As molding materials of the gasket 11, elastic rubber (e.g., butyl rubber, latex rubber, silicone rubber, and the like), and synthetic resin (e.g., styrene elastomer such as styrene-butadiene-styrene (SBS) elastomer and styrene-ethylene-butylene-styrene (SEBS) elastomer, olefin elastomer such as ethylene-α-olefin copolymer elastomer, and the like) are preferably used.

The plunger 12 is made of hard or semi-hard resin. In this example, the plunger 12 includes a gasket pressing portion 14 provided at a distal end and having a small disk shape, a pressing operation portion 15 provided at a proximal end and having a disk shape, and a shaft portion 16 having a cross shaped section and extending in the axial direction of the barrel 20 between the gasket pressing portion 14 and the pressing operation portion 15. The shaft portion 16 may be a columnar shaft. The columnar shaft may be a rod, a circular column, a polygonal column, a cylindrical tube, a square tube, and the like.

The plunger 12 is not connected to the gasket 11, is capable of contacting a proximal end portion of the gasket 11 at the gasket pressing portion 14, and can move the gasket 11 to the distal end side by the pressing after the abutment.

A distal end side portion (the gasket pressing portion 14 and a part in the distal end side of the shaft portion 16) of the plunger 12 is received in the barrel 20 (tubular body 21). The plunger 12 includes a stopper portion 17 that is provided at a part (shaft portion 16) to be received in the barrel 20, configured to abut on the grip 30 (stopper abutment portion 31) described in detail later, and configured to restrict the plunger 12 from being slipped from the barrel 20. More specifically, the stopper portion 17 has a disk shape having the outer shape (outer diameter) smaller than the inner shape (inner diameter) of the barrel 20 (tubular body 21) and larger than the outer shape (outer diameter) of the shaft portion 16 of the plunger 12.
Examples of constituent materials of the plunger 12 including a hard or semi-hard resin such as high-density polyethylene, polypropylene, polystyrene, or polyethylene terephthalate may be preferably used.

In the syringe 1, a drug (medicinal solution) 50 is stored (filled) in the barrel 20 (or, in a space formed between the seal cap 13 and the gasket 11).

Although the drug 50 to be stored is not particularly limited, examples of that include vitamins mainly as nutrients, sugars, electrolytes, organic acids, minerals, fat emulsions, amino acids, proteins, organ preparations, and the like. Furthermore, that include general anesthetics mainly as therapeutic agents, drugs for central nervous system such as antipyretic analgesic antiphlogistic agents and general cold preparations, drugs for peripheral nervous system such as local anesthetics and muscle relaxants, drugs for sensory organ such as ophthalmologic agents, metabolic drugs such as circulatory drugs, respiratory drugs, digestive drugs, urogenital anal drugs, hormonal agents, antibiotics, and diabetes drugs, antitumor drugs, allergic drugs, biological preparations such as antibacterial agents and antiviral agents, and prescription drugs such as distilled water and physiological saline. In addition, that include vaccines mainly as preventive agents, contrast agents mainly as diagnostic agents, and the like.

In these examples, many kinds of drugs are mainly composed of synthetic compounds of low molecular weight, synthetic compounds of middle molecular weight, biopharmaceuticals such as polypeptide preparations and protein preparations, and biologically derived pharmaceuticals such as blood preparations, but these substances can be used as drugs regardless of the use described above. In addition, substances that correspond to a molecular targeted drug such as an antibody can also be used as a drug. From the viewpoint of usability, the preferable dosage form of these drugs is a liquid preparation even in a drug having a dosage form of a solid preparation such as a powder preparation or a granule in addition to a liquid preparation.

In addition, as the drug 50, ophthalmic agents capable of being administered by puncture can be used. Specific target diseases for which such ophthalmic agents are used include choroidal neovascularization, age-related macular degeneration (both wet and dry), macular edema secondary to retinal vein occlusion (RVO) (including both branch retinal vein occlusion (bRVO) and central retinal vein occlusion (cRVO)), choroidal neovascularization secondary to pathologic myopia (PM), diabetic macular edema (DME), diabetic retinopathy, and proliferative retinopathy. Examples of the drug to be used include anti-VEGF antibodies used for the treatment of age-related macular degeneration, e.g., ranibizumab [trade name: LUCENTIS (registered trademark)], bevacizumab [trade name: AVASTIN (registered trademark)], and aflibercept [trade name: EYLEA (registered trademark)], and Conbercept known as VEGF-TrapEye (aflibercept intravitreal injection drug).

The structure of the grip 30 for a syringe barrel of the present invention will be described with reference to an example illustrated in FIGS. 1 to 6.

The grip 30 for a syringe barrel of this example includes a flange receiving portion 33 configured to receive the flange 22, and the side wall portion 32 extending in a distal end direction from the flange receiving portion 33 and configured to partially cover a proximal end portion of the tubular body 21 adjacent to the flange 22. The side wall portion 32 includes a fitting portion 34 configured to be fitted to the proximal end portion of the tubular body 21, and an airflow portion (openings 35) configured to allow a sterilization gas to flow to an outer surface of the tubular body 21 covered by the side wall portion 32.

In the example, the side wall portion 32 is a tubular portion (short substantially semi-cylindrical portion) having an insertion opening for the proximal end portion of the tubular body 21.

Specifically, the flange receiving portion 33 of the grip 30 constitutes a proximal end side portion of the grip 30. On the flange receiving portion 33, a pair of finger hook portions 36 and 36 is formed projecting outward (in the direction orthogonal to the axial direction of the barrel 20). The finger hook portions 36 and 36, being a couple, project in opposite directions from each other along the direction orthogonal to the axial direction of the barrel 20. The pair of finger hook portions 36 and 36 extends outward further than the flange 22 of the barrel 20. An operator can use the pair of finger hook portions 36 and 36 to hook fingers when operating the syringe 1.

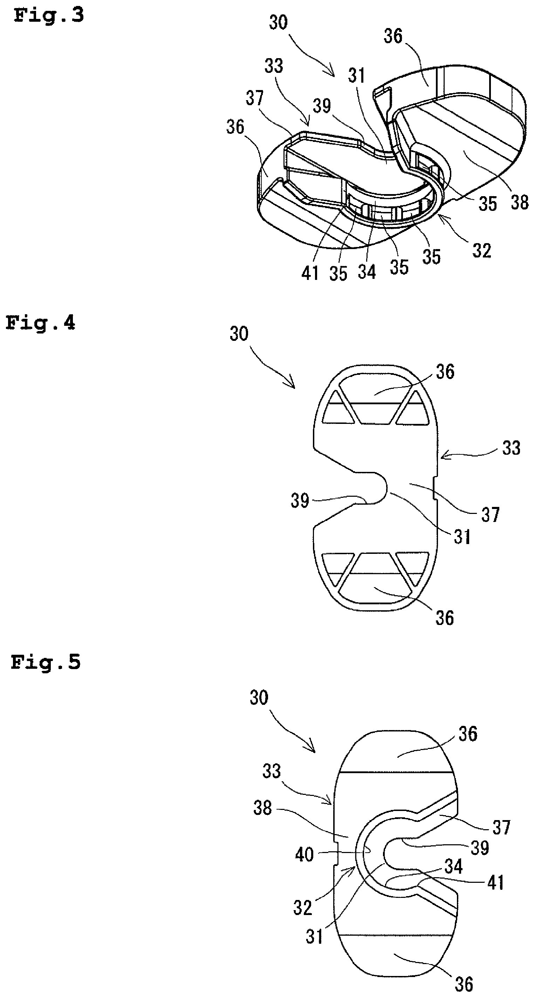

The flange receiving portion 33 includes an upper plate portion 37 and a lower plate portion 38 that sandwich the flange 22. As illustrated in FIG. 4, a slit (opening) 39 is formed in the upper plate portion 37 of the flange receiving portion 33. The slit 39 is formed such that the shaft portion 16 of the plunger 12 can enter the slit 39. A part of an inner edge portion of the slit 39 is a stopper abutment portion 31 that abuts on the stopper portion 17 of the plunger 12. The stopper abutment portion 31 has the inner shape (inner diameter) larger than the outer shape (outer diameter) of the shaft portion 16 of the plunger 12 and smaller than the outer shape (outer diameter) of the stopper portion 17 of the plunger 12. As a result, the slit 39 allows the shaft portion 16 of the plunger 12 to pass through the stopper abutment portion 31, and abuts on the stopper portion 17 of the plunger 12. The plunger 12 received in the barrel 20 abuts on the stopper abutment portion 31 of the grip 30 at the stopper portion 17, so that the plunger 12 is restricted from being slipped from the barrel 20.

As illustrated in FIG. 5, a slit (opening) 40 is formed in the lower plate portion 38 of the flange receiving portion 33. An inner edge portion of the slit 40 is integrated (connected) with the side wall portion 32 described in detail later.

As illustrated in FIG. 2, the interval between the upper plate portion 37 and the lower plate portion 38 of the flange receiving portion 33 is slightly larger than the thickness of the flange 22 of the barrel 20. As a result, a slight gap through which the sterilization gas can flow is formed between the upper surface (surface on the proximal end side) and/or the lower surface (surface on the distal end side) of the flange 22 and the upper plate portion 37 and/or the lower plate portion 38 of the flange receiving portion 33.

The grip 30 includes the side wall portion 32 extending in the distal direction, specifically, toward the axial distal end side from the flange receiving portion 33. The side wall portion 32 is integrated (connected) with the inner edge portion of the slit 40 in the lower plate portion 38 of the flange receiving portion 33 at the proximal end. The side wall portion 32 is formed with an attachment (for insertion for the proximal end portion of the tubular body) opening (attachment slit) 41 extending over the entire length in the axial direction, and the proximal end portion of the barrel 20 (tubular body 21) is partially covered in a part other than the opening 41. The side wall portion 32 excluding the opening 41 extends in the circumferential direction. In other words, the side wall portion 32 is obtained by cutting out a part of the cylindrical member in the circumferential direction as the attachment slit 41, and is formed such that a cross section by a plane orthogonal to the axial direction has a C shape. In the example, the side wall portion 32 is a tubular portion (short substantially semi-cylindrical portion) having an insertion opening for the proximal end portion of the tubular body 21.

The attachment slit 41 in a natural state where the side wall portion 32 is not elastically deformed has an opening width smaller than the outer diameter of the tubular body 21 of the barrel 20. In the process of attaching the grip 30 to the barrel 20, the attachment slit 41 is elastically deformed by the barrel 20 to be widened. When the grip 30 has been attached to the barrel 20, the attachment slit 41 returns to the original opening width by the elastic restoring force. As a result, the grip 30 is firmly attached to the barrel 20.

In the side wall portion 32, a part to be in direct contact with the outer surface of the tubular body 21 of the barrel 20 forms the fitting portion 34. In other words, the fitting portion 34 refers to an inner surface of the side wall portion 32 to be in direct contact with the outer surface of the tubular body 21 and a part of the side wall portion 32 corresponding to such an inner surface. In the present example, a part in the side wall portion 32 where the openings 35 (described in detail later) are not formed and an inner surface of the part form the fitting portion 34.

The plurality of openings 35 are formed in the side wall portion 32. These openings 35 constitute an airflow portion. In the grip 30, the airflow portion is formed by the openings 35 formed on the side wall portion 32. In the present example, the six openings 35 like rectangular holes (windows) are formed in the side wall portion 32. Each opening 35 penetrates the side wall portion 32 in the radial direction (direction orthogonal to the axial direction). Through the part where the openings 35 are formed, the outer surface of the tubular body 21 of the barrel 20 is exposed to the outside.

A contact area between the side wall portion 32 of the grip 30 for a syringe barrel and the proximal end portion of the tubular body 21 when the grip 30 is attached to the syringe barrel 20 is preferably 40% or less of the area of the proximal end portion of the tubular body 21 covered by the side wall portion 32. With this configuration, sterilization is ensured.

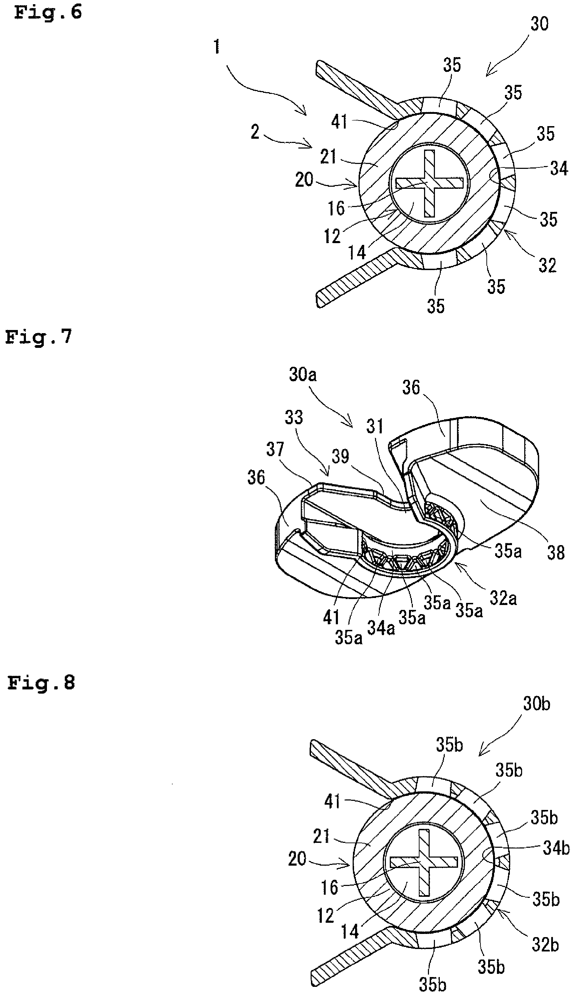

In the present example, as illustrated in FIG. 6, in the side wall portion 32, the side wall portion 32 (fitting portion 34) where the openings 35 are not formed is formed to be thinner toward the inside in the cross section on a plane orthogonal to the axial direction. For this reason, the area of the contact portion between the fitting portion 34 and the barrel 20 is small. The contact area between the side wall portion 32 of the grip 30 for a syringe barrel and the proximal end portion of the tubular body 21 in the example illustrated in FIGS. 1 to 6 is 50% or less of the area of the proximal end portion of the tubular body 21 covered by the side wall portion 32.

In the side wall portion 32, 5 to 10 openings 35 like rectangular holes are preferably formed. In addition, the area of the part (fitting portion 34) where the inner surface of the side wall portion 32 and the outer surface of the tubular body 21 are in contact with each other is preferably 10 to 50%, and particularly preferably 10 to 40%, as compared with the case where the openings 35 are not formed (the area of the inner surface of the entire side wall portion 32). In addition, the length of the side wall portion 32 in the axial direction is preferably 0.17 to 3.4 mm, and particularly preferably 0.34 to 1.7 mm.

It is desirable that none of the openings 35 be opened at the distal end portion (end surface) of the side wall portion 32. As a result, the strength of the distal end portion of the side wall portion 32 is maintained, the grip 30 can be firmly attached to the barrel 20 while reducing the area of the fitting portion 34, and rattling or the like of the grip 30 can be suppressed.

As a constituent material of the grip 30, a hard or semi-hard resin, e.g., polyolefin such as polyethylene and polypropylene, polystyrene, polyamide, polycarbonate, polyvinyl chloride, poly-(4-methylpentene-1), acrylic resin, acrylonitrile-butadiene-styrene copolymer, polyester such as polyethylene terephthalate, cyclic polyolefin polymer, and cyclic olefin copolymer is preferably used.

As the constituent material of the grip 30, a material having a thermal expansion coefficient (linear thermal expansion coefficient) larger than that of the barrel 20 may be used. As a result, when the syringe 1 is heated in a surface sterilization (gas sterilization) step described later, the inner surface (inner diameter) of the grip 30 expands more greatly than the outer surface (outer diameter) of the barrel 20 (tubular body 21), a slight gap is formed between a part of the inner surface (fitting portion 34) of the grip 30 and the outer surface of the tubular body 21, and the sterilization gas enters the gap, whereby the sterilizability of the syringe 1 can be improved.

A manufacturing method (manufacturing process) of such a syringe 1 will be described.

First, the barrel 20 attached to the seal cap 13 in the nozzle portion 23 of the barrel 20 is sterilized. The sterilization method adopted here is not particularly limited, and for example, an autoclave sterilization method using an autoclave (high temperature steam sterilization method), a surface sterilization method using a sterilization gas such as hydrogen peroxide or EOG (gas sterilization method), a radiation sterilization method by radiation irradiation such as γ ray or electron radiation, and the like can be used. Here, as an example, autoclave sterilization is performed.

Next, under a sterile environment, the barrel 20 having been sterilized is aseptically filled with the drug 50 that has already been subjected to sterilization treatment (for example, filtration sterilization or the like). In this example, the drug 50 for ophthalmic use, which is relatively sensitive to heat, is filled. After the drug50 is filled, the gasket 11 is inserted into the barrel 20. Insertion of the gasket 11 (plugging) can be performed in a depressurized state.

Next, a part in the distal end side of the plunger 12 is received in the barrel 20, and the grip 30 is attached to the barrel 20. This operation can also be performed in a place under an unsterilized environment.

Next, the syringe 1 with the grip 30 attached to the barrel 20 is sterilized. In the present example, since the drug 50 stored in the barrel 20 is a drug for ophthalmic use that is relatively sensitive to heat, surface sterilization using a sterilization gas (here, NO₂ (nitrogen dioxide)) is performed (NO₂ sterilization).

As described above, the syringe 1 can be aseptically manufactured. The grip 30 (side wall portion 32) of the present example has the plurality (here, six) of the openings 35. Through each opening 35, the sterilization gas is brought into contact with the outer surface of the proximal end portion of the barrel 20 (tubular body 21) partially covered by the side wall portion 32. As a result, the syringe 1 including the barrel 20 to which the grip 30 has been attached (barrel assembly 2) is more reliably sterilized.

In the present example, in the side wall portion 32, the side wall portion 32 (fitting portion 34) where the openings 35 are not formed is formed to be thinner toward the inside in the cross section on a plane orthogonal to the axial direction. As a result, the sterilization gas can be brought into contact with a wider area of the outer surface of the barrel 20 (tubular body 21) and the inner surface of the grip 30, while the strength of the fitting portion 34 is maintained. The shape of the fitting portion 34 in the side wall portion 32 is appropriately set based on molding conditions or the like.

In addition, the sterilization gas can enter a gap inevitably formed between the inner surface of the fitting portion 34 and the outer surface of the tubular body 21 from a peripheral portion of the fitting portion 34 in the side wall portion 32. In the grip 30 of the present example, since the openings 35 are formed in the side wall portion 32, the sterilization gas can enter between the inner surface of the fitting portion 34 and the outer surface of the tubular body 21 also from the peripheral portions of the openings 35.

As a result, the sterilization gas can be brought into contact with a wider area of the outer surface of the barrel 20 (the tubular body 21) and the inner surface of the grip 30, so that sterilization can be performed more reliably. When focusing on such an effect, forming a large number of relatively small openings in order to increase the peripheral edge length of the openings (the total length of the peripheral edge portions of the openings) with respect to the opening area (the total area of the openings) is also effective. Specifically, it is conceivable that a large number of through holes are formed as relatively small openings.

The shape of the opening formed as the airflow portion in the side wall portion is not limited to the rectangular hole described above, and may be, for example, a circular, triangular, or other polygonal hole (window). In a grip 30a illustrated in FIG. 7, a plurality of openings 35a like triangular holes are formed in a side wall portion 32a in a vertically inverted manner. In this case, the structure of a part in the side wall portion 32a other than the part where the openings 35a are formed is what is called a truss structure. As a result, when the opening area is the same, the strength of the side wall portion 32a can be further increased as compared with the case where openings like rectangular holes are formed.

When the opening formed in the side wall portion is formed by the openings 35a like triangular holes providing the side wall portion 32a with the truss structure, six to 20 openings 35a are preferably formed. The contact area between the side wall portion 32a of the grip 30a for a syringe barrel and the proximal end portion of the tubular body 21 is preferably 10 to 40% of the area of the proximal end portion of the tubular body 21 covered by the side wall portion 32a. In other words, the contact area between the side wall portion 32a of the grip for a syringe barrel and the proximal end portion of the tubular body 21 when the grip is attached to the syringe barrel (the area of the part where the inner surface of the side wall portion 32a and the outer surface of the tubular body 21 are in contact with each other) is preferably 10 to 40% of the area of the proximal end portion of the tubular body 21 covered by the side wall portion 32a (the area of the inner surface of the entire side wall portion 32a when the openings are not formed).

In addition, as in a grip 30b illustrated in FIG. 8, in a side wall portion 32b, an inner surface of the side wall portion 32b where openings 35b are not formed excluding both axial end portions (a part excluding both end portions of the fitting portion, in other words, an intermediate part of the fitting portion) may be formed so as to be isolated from the outer surface of the tubular body 21. As a result, the sterilization gas can be brought into contact with a wider area of the outer surface of the barrel 20 (tubular body 21) and the inner surface of the grip 30b, while the attachment force (fitting force) of the grip 30b at both axial end portions of the side wall portion 32b (fitting portion 34b) to the tubular body 21 is maintained.

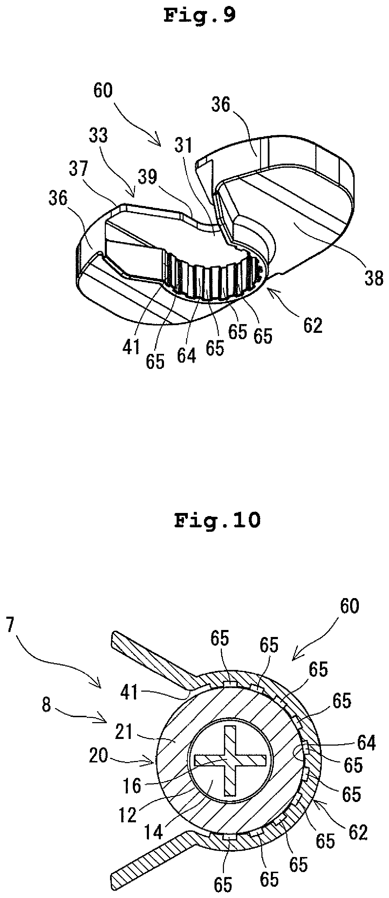

FIGS. 9 to 18 respectively illustrate barrel assemblies including grips for a syringe barrel and syringes according to other embodiments of the present invention. The forms of airflow portions in these examples are different from that of the grip 30 of the example (the syringe 1 and the barrel assembly 2) described above. In these examples, unless otherwise specified, the same names and reference numerals are used for configurations common to the example described above and the other examples, and detailed description thereof is omitted.

In the syringes and the barrel assemblies of the present invention illustrated in FIGS. 9 to 18, in the grip, the airflow portion is provided on the inner surface of the side wall portion, and the airflow portion is formed by a non-contact portion between the inner surface of the side wall portion and the outer surface of the tubular body. Such an airflow portion reduces an area of a contact portion (fitting portion) between the inner surface of the side wall portion and the outer surface of the tubular body, and increases a contact area between the sterilization gas and the inner surface of the side wall portion and the outer surface of the tubular body covered by the side wall portion.

In a syringe 7 and a barrel assembly 8 of the present invention illustrated in FIGS. 9 and 10, the airflow portion of a grip 60 for a syringe barrel (hereinafter simply referred to as the grip 60) is formed by a plurality of recesses 65 formed on an inner surface of a side wall portion 62.

Specifically, on the inner surface of the side wall portion 62 of the grip 60, a plurality (here, 10) of recesses 65 that have a substantially rectangular cross section on a plane orthogonal to the axial direction and extend in the axial direction are formed. Each recess 65 is formed over the entire length in the axial direction of the side wall portion 62. In other words, each recess 65 is open at both axial end portions of the side wall portion 62. The inner surface of the side wall portion 62 and the outer surface of the tubular body 21 are not in contact with each other at the part where the recesses 65 are formed.

In the present example, parts in the side wall portion 62 where the recesses 65 are not formed and the inner surface of the side wall portion 62 form fitting portions 64 configured to be fitted to the proximal end portion of the tubular body 21. The fitting portions 64 each have a substantially rectangular cross section on a plane orthogonal to the axial direction.

On the grip 60 (side wall portion 62) of the present example, the plurality (here, 10) of recesses 65 are formed. The sterilization gas is brought into contact with the inner surface of the side wall portion 62 and the outer surface of the proximal end portion of the barrel 20 (tubular body 21) partially covered by the side wall portion 62, through each recess 65. As a result, the syringe 7 including the barrel 20 to which the grip 60 has been attached (barrel assembly 8) is more reliably sterilized. The number of recesses 65 (fitting portions 64) is preferably 3 to 30, and particularly preferably 10 to 20.

In addition, in the present example, the recesses 65 formed on the grip 60 (side wall portion 62) are open at both axial end portions of the side wall portion 62, so that the sterilization gas easily flows (enters or passes) through the recesses 65. In addition, in this example, the width (circumferential dimension) of each recess 65 formed on the grip 60 (side wall portion 62) is wide at the distal end of the side wall portion 62 (the lower end of the side wall portion 62 in FIG. 9) and is narrowed toward the proximal end side. As a result, the sterilization gas easily comes in. In addition, the width (circumferential dimension) of each fitting portion 64 formed on the grip 60 (side wall portion 62) is narrow at the distal end of the side wall portion 62 (the lower end of the side wall portion 62 in FIG. 9) and is widened toward the proximal end side. As a result, an outer tube is reliably held at the proximal end side of the side wall portion 62.

In the side wall portion 62, three to 10 recesses 65 are preferably formed on the inner surface of the side wall portion 62. In addition, the contact area between the side wall portion 62 of the grip 60 for a syringe barrel and the proximal end portion of the tubular body 21 is preferably 0.1 to 40% of the area of the proximal end portion of the tubular body 21 covered by the side wall portion 62. In other words, the contact area between the side wall portion 62 of the grip for a syringe barrel and the proximal end portion of the tubular body 21 when the grip is attached to the syringe barrel (the area of the part where the inner surface of the side wall portion 62 and the outer surface of the tubular body 21 are in contact with each other) is preferably 0.1 to 40% of the area of the proximal end portion of the tubular body 21 covered by the side wall portion 62 (the area of the inner surface of the entire side wall portion 62 when the recesses are not formed).

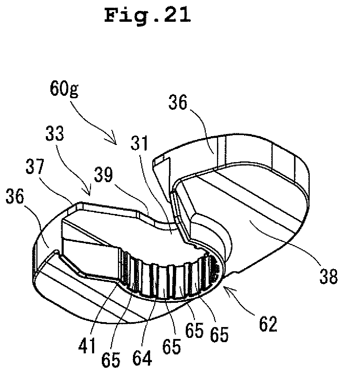

In addition, a grip 60g illustrated in FIG. 21 has the same configuration as that of the grip 60 illustrated in FIG. 9, and both grips have the same number of recesses 65 formed on the side wall portion 62 (10 pieces), but the width (circumferential dimension) of each recess 65 of grip 60g is wider (in other words, the width of each fitting portion 64 is narrower) than the grip 60. As a result, in the grip 60g illustrated in FIG. 21, the contact area between the side wall portion 62 of the grip 60g for a syringe barrel and the proximal end portion of the tubular body 21 is smaller than that of the grip 60 illustrated in FIG. 9 described above, and is 30% of the area of the proximal end portion of the tubular body 21 covered by the side wall portion 62.

In addition, also in the grip 60g of the example illustrated in FIG. 21, the width of each recess 65 formed on the grip 60g (side wall portion 62) is wide at the distal end of the side wall portion 62 (the lower end of the side wall portion 62 in FIG. 21) and is narrowed toward the proximal end side. As a result, the sterilization gas easily comes in. In addition, the width of each fitting portion 64 formed on the grip 60g (side wall portion 62) is narrow at the distal end of the side wall portion 62 (the lower end of the side wall portion 62 in FIG. 21) and is widened toward the proximal end side. As a result, an outer tube is reliably held at the proximal end side of the side wall portion 62.

The cross-sectional shape on a plane orthogonal to the axial direction of each recess formed on the side wall portion as the airflow portion is not limited to a substantially rectangle as described above, and may be various shapes such as a triangular shape, a trapezoidal shape, another polygonal shape, and a crescent shape. It is desirable that the recesses be formed so as to have a trapezoidal or triangular cross section on a plane orthogonal to the axial direction such that the contact area between the inner surface of the side wall portion (fitting portion) and the outer surface of the tubular body is reduced (or, such that the shape of the fitting portion is narrowed radially inward in the cross section on a plane orthogonal to the axial direction). In addition, the recesses forming the airflow portion preferably extend parallel to the axial direction, but may extend obliquely or extend spirally with respect to the axis.

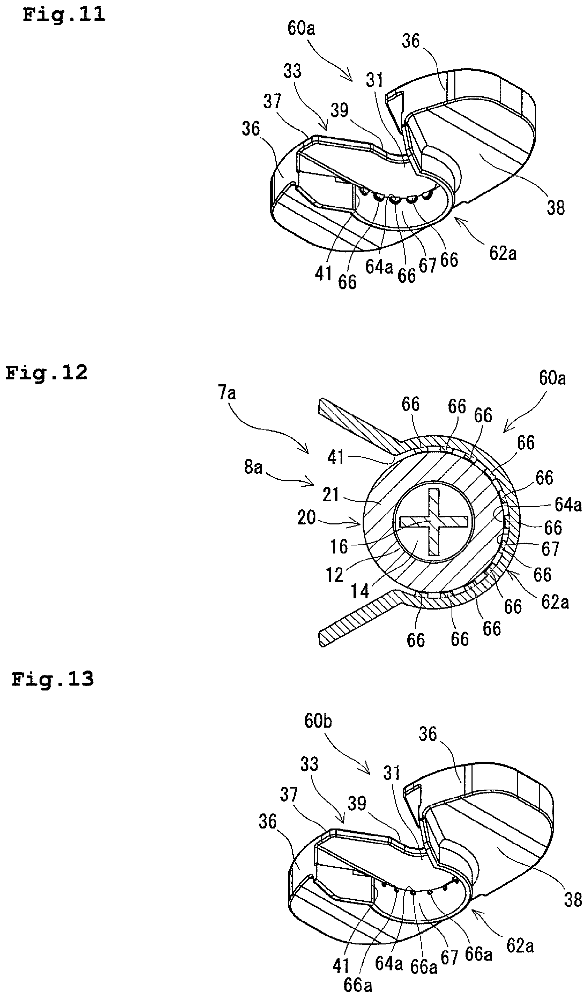

FIGS. 11 and 12 illustrate the barrel assembly including the grip for a syringe barrel and the syringe according to yet another embodiment of the present invention. The form of airflow portion in the present example is different from that of the grip 60 of the example (the syringe 7 and the barrel assembly 8) described above.

In a grip 60a of a syringe 7a and a barrel assembly 8a of the present example, the airflow portion is formed by a part (inter-protrusion portion 67) between a plurality of protrusions 66 formed on an inner surface of a side wall portion 62a.

Specifically, the plurality (here, 11) of protrusions 66 protruding radially inward are formed on the inner surface of the side wall portion 62a of the grip 60a. The protrusions 66 each have a substantially semicircular shape as viewed from the radially inner side of the side wall portion 62a (as viewed in the radial direction), and are formed on the inner surface of the proximal end portion (upper end portion) of the side wall portion 62a, the protrusions 66 being arranged on the same circle. Upper surfaces of the protrusions 66 are substantially flush with an upper surface of the lower plate portion 38. In the present example, inner surfaces of the protrusions 66 and a part of the side wall portion 62a where the protrusions 66 are formed are fitting portions 64a configured to be fitted to the proximal end portion of the tubular body 21. An inner surface of a part (inter-protrusion portion 67) of the side wall portion 62a where the protrusions 66 are not formed is not in contact with the outer surface of the tubular body 21. In the grip 60 a, as the airflow portion, the inter-protrusion portion 67 which is a part not in contact with the outer surface of the tubular body 21 is formed on the inner surface of the side wall portion 62a.

On the grip 60a (side wall portion 62a) of the present example, the plurality (here, 11) of protrusions 66 are formed. The sterilization gas is brought into contact with the inner surface of the side wall portion 62a and the outer surface of the proximal end portion of the barrel 20 (tubular body 21) partially covered by the side wall portion 62a, through the inter-protrusion portion 67 that is formed between the protrusions 66 not in contact with the outer surface of the tubular body 21. As a result, sterilizability of the syringe 7a including the barrel 20 to which the grip 60a has been attached (barrel assembly 8a) can be further improved.

In addition, the inter-protrusion portion 67 formed on the grip 60a (side wall portion 62a) is open to the outside at both axial end portions and both circumferential ends of the side wall portion 62a, so that the sterilization gas easily flows (enters or passes) through the inter-protrusion portion 67.

In the side wall portion 62a, 3 to 20 protrusions 66 are preferably formed on the inner surface of the side wall portion 62a. In addition, the contact area between the side wall portion 62a of the grip 60a for a syringe barrel and the proximal end portion of the tubular body 21 is preferably 0.1 to 40% of the area of the proximal end portion of the tubular body 21 covered by the side wall portion 62a. In other words, the area of a part (fitting portion 64a) where the inner surface of the side wall portion 62a (inner surfaces of the protrusions 66) and the outer surface of the tubular body 21 are in contact with each other is preferably 0.1 to 40% of the area of the part where the inner surface of the side wall portion 62 and the outer surface of the tubular body 21 are in contact with each other when the protrusions are not formed. In this example, the contact area between the side wall portion 62a of the grip 60a for a syringe barrel and the proximal end portion of the tubular body 21 is 14% of the area of the proximal end portion of the tubular body 21 covered by the side wall portion 62a.

The shape of each protrusion (as viewed in the radial direction) formed on the side wall portion is not limited to the exemplified semicircular shape, and may be, for example, a rectangular shape or another polygonal shape. In addition, the shape may be a conical shape, a triangular pyramid shape, another polygonal pyramid shape, a truncated cone shape, a triangular frustum shape, and another polygonal frustum shape that have a cross-sectional area gradually decreasing radially inward.

In addition, the protrusions formed on the grip (side wall portion) may have the form illustrated in FIG. 13. In a grip 60b of the example illustrated in FIG. 13, projections 66a having a granular shape are formed on the inner surface of the grip (side wall portion). As a result, the contact area (area of the fitting portion 64a) between the protrusions 66a (inner surface of the side wall portion 62a) and the outer surface of the tubular body 21 can be significantly reduced.

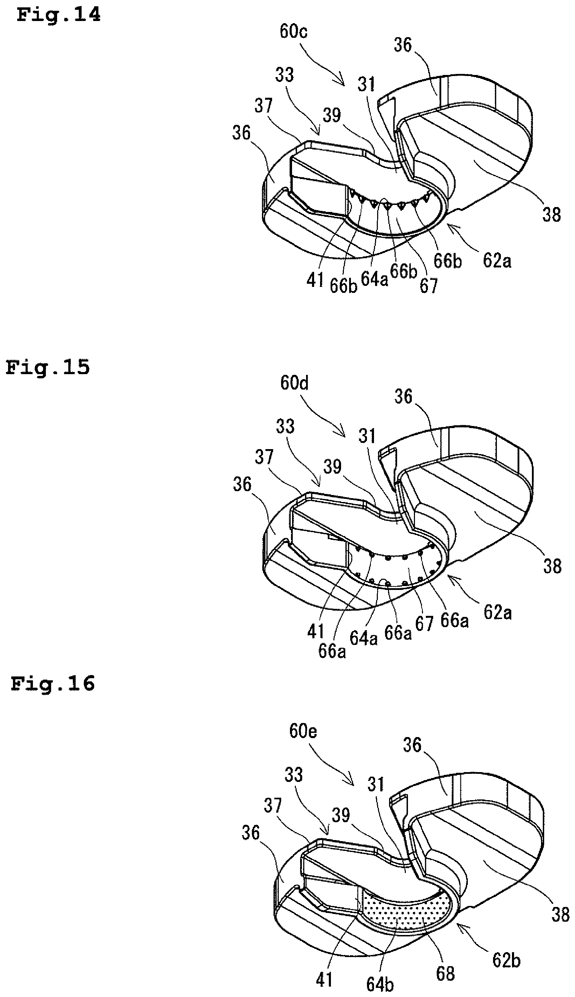

In addition, the protrusions formed on the grip (side wall portion) may have the form illustrated in FIG. 14. Regarding a grip 60c of the example illustrated in FIG. 14, protrusions 66b having a substantially triangular pyramid (substantially tetrahedral) shape formed on an inner peripheral edge portion at a proximal end of the side wall portion 62a in the grip 60c are further illustrated in FIG. 14. As a result, the contact area (area of the fitting portion 64a) between the protrusions 66b (inner surface of the side wall portion 62a) and the outer surface of the tubular body 21 can be significantly reduced.

The protrusions are not limited to those provided on the same circle as exemplified, and may be provided at arbitrary positions. In addition, for example, the protrusions may be provided over two or more rows on circles having different axial positions, or may be provided in two rows in an alternate (staggered) manner. A grip 60d illustrated in FIG. 15 includes protrusions 66a provided over two rows on circles (at the proximal end portion and the distal end portion of the side wall portion 62a) having different axial positions.

FIGS. 16 to 18 illustrate the barrel assembly including the grip for a syringe barrel and the syringe according to yet another embodiment of the present invention. The form of airflow portion in the present example is different from that of the grip 60 of the examples (the syringes 7, 7a and the barrel assemblies 8, 8a) described above.

In a grip 60e of a syringe 7b and a barrel assembly 8b of the present example, the airflow portion is formed by a rough surface portion 68 formed on an inner surface of a side wall portion 62b.

Specifically, the rough surface portion 68 is formed on the entire inner surface of the side wall portion 62b of the grip 60e (indicated by dot hatching in FIG. 16). The rough surface portion 68 is formed by publicly known surface processing such as embossing or shot blasting. In the present example, a mold (injection molding mold) for molding the grip 60e is roughened in advance and the rough surface portion 68 is formed by emboss transfer (emboss processing).

The rough surface portion 68 formed on the inner surface of the side wall portion 62b is preferably formed such that the sterilization gas can enter the rough surface portion 68 in a state where the grip 60e is attached to the barrel 20 (tubular body 21).

As the degree of surface roughness of the rough surface portion 68, an embossed portion in which a plurality of recessed portions intersecting each other and having a thin grooved shape with a depth of about 5 to 1000 µm are formed is preferable.

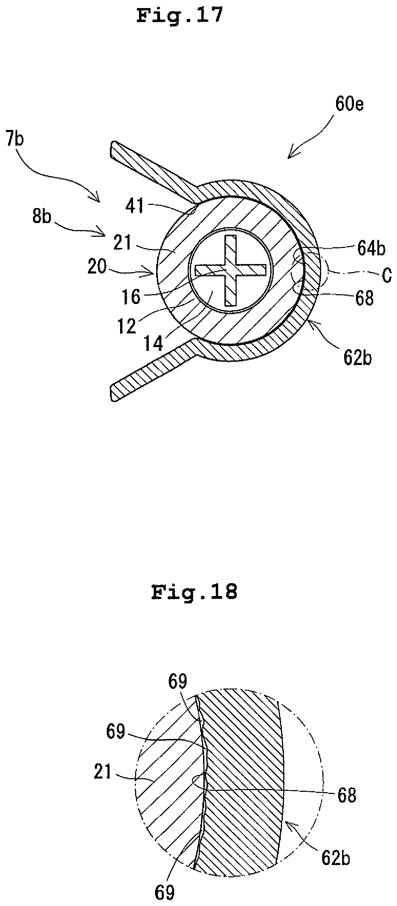

As a result, a non-contact portion between the inner surface of the side wall portion 62b and the outer surface of the tubular body 21 is formed at a part where the rough surface portion 68 is formed. FIG. 18 schematically illustrates that a portion 69 is formed on the rough surface portion 68 such that the sterilization gas can enter the portion 69 (unevenness of the rough surface portion 68 is significantly emphasized).

On the grip 60e (side wall portion 62b) of the present example, the rough surface portion 68 is formed. The sterilization gas is brought into contact with the inner surface of the side wall portion 62b and the outer surface of the proximal end portion of the barrel 20 (tubular body 21) partially covered by the side wall portion 62, through the rough surface portion 68. As a result, the syringe 7b including the barrel 20 (barrel assembly 8b) to which the grip 60e has been attached is more reliably sterilized.

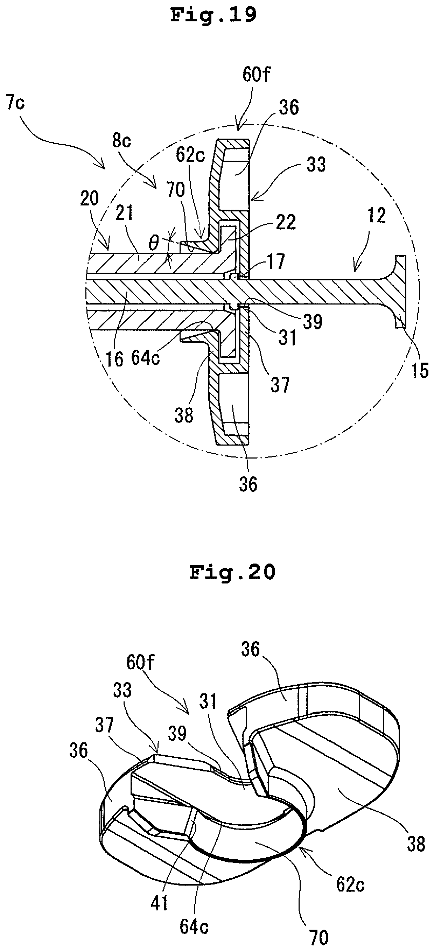

FIGS. 19 and 20 illustrate the barrel assembly including the grip for a syringe barrel and the syringe according to yet another embodiment of the present invention. FIG. 19 is an enlarged sectional view of the proximal end portion of the syringe according to yet another example of the present invention. FIG. 20 is a perspective explanatory view of the grip for a syringe barrel used in the syringe in FIG. 19. A syringe 7c in the present example includes a grip 60f having an airflow portion with a form different from that of the grip 60 of the example (the syringe 7 and the barrel assembly 8) described above.

In the grip 60f of the syringe 7c and the barrel assembly 8c of this example, an airflow portion 70 is formed by a tapered portion formed on an inner surface of a side wall portion 62c. In addition, a fitting portion 64c is formed of a small diameter portion (a part having a small inner diameter) at a base end of the tapered portion. The side wall portion 62c is a tapered portion in which the inner diameter increases toward the distal end, and the entire outer diameter is substantially the same. The side wall portion 62c therefore becomes thinner toward the distal end. In a part excluding the fitting portion 64c, the proximal end portion of the barrel 20 (tubular body 21) partially covered by the side wall portion 62c is not in contact with the side wall portion 62c. In this part, therefore, the outer surface of the barrel 20 and the tapered portion (airflow portion 70) of the side wall portion 62c come into contact with the sterilization gas. As a result, sterilizability of the syringe 7c including the barrel 20 to which the grip 60f has been attached (barrel assembly 8c) can be further improved.

In the present example, in order to further reduce the area of the fitting portion 64c and improve the sterilizability, as illustrated in FIG. 19, the inner surface (tapered portion 70) of the side wall portion 62c is inclined so as to be separated from the outer surface of the tubular body 21 at a predetermined angle (θ) toward the distal end side. The area of the part (fitting portion 64c) where the inner surface of the side wall portion 62c and the outer surface of the tubular body 21 are in contact with each other is preferably 0.1 to 50%, and particularly preferably 10 to 40%, as compared with the case where the inner surface of the entire side wall portion 62c is not the tapered portion. In addition, the taper angle (θ in FIG. 19) in the side wall portion 62c is preferably 0.2 degrees or more, and particularly preferably 0.5 degrees or more. In addition, the fitting portion 64c may include a recess or a groove extending in the axial direction and communicating the airflow portion 70 and the flange receiving portion 33. By providing such a recess or groove, the flow of the sterilization gas is further improved.

The inner surface of the side wall portion as the tapered portion is not limited to the inclined surface linearly separated from the outer surface of the tubular body 21 as in the present example, and may be, for example, a convex inward, curved surface or a concave outward, curved surface. In addition, the tapered portion may be provided from the axial center portion of the side wall portion toward the distal end side.

In the grip for a syringe barrel, and the barrel assembly and the syringe including such a grip for a syringe barrel of the present invention, the airflow portion of the grip is not limited to one that forms only one of the above-described opening and non-contact portion (recesses, inter-protrusion portion, rough surface portion, and tapered portion), and the airflow portion may be formed by appropriately combining these configurations.

### INDUSTRIAL APPLICABILITY

A grip for a syringe barrel of the present invention is defind in claim 1.

With the grip for a syringe barrel according to claim 1, the sterilization gas can be brought into contact with a wider range of the outer surface of the syringe barrel, and the syringe barrel attached to the syringe barrel grip is more reliably sterilized.

In addition, the above-described aspect may be as follows, forming only insofar part of the invention as corresponding to the subject-matter of the claims.
(2) The grip for a syringe barrel according to aspect (1), in which the side wall portion is a tubular portion having an insertion opening for the proximal end portion of the tubular body.
(3) The grip for a syringe barrel according to (1) or (2), in which the airflow portion is formed by a plurality of openings formed in the side wall portion.
(4) The grip for a syringe barrel according to (1) or (2), in which the airflow portion is provided on an inner surface of the side wall portion, and the airflow portion is formed by a non-contact portion between the inner surface of the side wall portion and the outer surface of the tubular body.
(5) The grip for a syringe barrel according to (4), in which the airflow portion reduces an area of a contact portion between the inner surface of the side wall portion and the outer surface of the tubular body, and increases a contact area between sterilization gas and the inner surface of the side wall portion and the outer surface of the tubular body covered by the side wall portion.
(6) The grip for a syringe barrel according to (4) or (5), in which the airflow portion is formed by a plurality of recesses formed on the inner surface of the side wall portion.
(7) The grip for a syringe barrel according to (4) or (5), in which the airflow portion is formed by a part between a plurality of protrusions formed on the inner surface of the side wall portion.
(8) The grip for a syringe barrel according to (4) or (5), in which the airflow portion is formed by a rough surface portion formed on the inner surface of the side wall portion.
(9) The grip for a syringe barrel according to (4) or (5), in which the airflow portion is formed by a tapered portion formed on the inner surface of the side wall portion.
(10) The grip for a syringe barrel according to any one of (1) to (9), in which a contact area between the side wall portion of the grip for a syringe barrel and the proximal end portion of the tubular body when the grip is attached to the syringe barrel is 40% or less of the area of the proximal end portion of the tubular body covered by the side wall portion.

A barrel assembly of the present invention is as follows.
(11) A barrel assembly including: a syringe barrel including a tubular body and a flange provided at a proximal end of the tubular body and projecting outward; and the grip for a syringe barrel according to any one of (1) to (10) attached to the barrel.

With the barrel assembly described above, the sterilization gas can be brought into contact with a wider range of the outer surface of the syringe barrel, and the barrel assembly is more reliably sterilized.

A syringe of the present invention is as follows.
(12) A syringe including: the barrel assembly according to (11); a gasket slidably received in the barrel; and a plunger configured to move the gasket.

With the syringe described above, the sterilization gas can be brought into contact with a wider range of the outer surface of the syringe barrel, and the syringe is more reliably sterilized.

In addition, the above-described aspect may be as follows.
(13) The syringe according to (12), in which a distal end side portion of the plunger is received in the barrel, and the plunger includes a stopper portion that is provided at a part to be received in the barrel, configured to abut on the grip, and configured to restrict the plunger from being slipped from the barrel.
(14) The syringe according to (12) or (13), in which the plunger is not connected to the gasket and includes a gasket pressing portion that is provided at a distal end and is capable of contacting a proximal end portion of the gasket.
(15) The syringe according to any one of (12) to (14), in which a drug is stored in the barrel.

## Claims

1. A grip (30, 30a-b, 60, 60c-g) for a syringe barrel (20) to be attached to a syringe barrel (20) that includes a tubular body (21), and a flange (22) provided on the tubular body (21) and projecting outward, wherein
the grip (30, 30a-b, 60, 60c-g) comprises:
a flange receiving portion (33) configured to receive the flange (22); and a side wall portion (32, 32a-b, 62, 62a-c) extending in a distal end direction from the flange receiving portion (33) and configured to partially cover a proximal end portion of the tubular body (21) adjacent to the flange (22), wherein
the side wall portion (32, 32a-b, 62, 62a-c) includes a fitting portion (34, 34b, 64, 64a, 64c) configured to be fitted to the proximal end portion of the tubular body (21), and an airflow portion (70) configured to allow sterilization gas to flow to an outer surface of the tubular body (21) covered by the side wall portion (32, 32a-b, 62, 62a-c),
the side wall portion (32, 32a-b, 62, 62a-c) is a tubular portion having an attachment slit (41) for the proximal end portion of the tubular body(21), the attachment slit (41) has an opening width smaller than an outer diameter of the tubular body (21) of the barrel (20), in the process of attaching the grip (30, 30a-b, 60, 60c-g) to the barrel (20), the attachment slit (41) is elastically deformed by the barrel (20) to be widened, when the grip (30, 30a-b, 60, 60c-g) has been attached to the barrel (20), the grip (30, 30a-b, 60, 60c-g) is firmly attached to the barrel (20) by the elastic restoring force,
the airflow portion (70) is provided on an inner surface of the side wall portion (32, 32a-b, 62, 62a-c), the airflow portion (70) is formed by a non-contact portion between the inner surface of the side wall portion (32, 32a-b, 62, 62a-c) and the outer surface of the tubular body (21), and the airflow portion (70) is formed by a plurality of recesses (65) formed on the inner surface of the side wall portion (32, 32a-b, 62, 62a-c) or a part between a plurality of protrusions (66, 66a-b) formed on the inner surface of the side wall portion (32, 32a-b, 62, 62a-c) or rough surface portion (68) formed on the inner surface of the side wall portion (32, 32a-b, 62, 62a-c).

2. The grip (30, 30a-b, 60, 60c-g) for a syringe barrel (20) according to claim 1, wherein the airflow portion (70) reduces an area of a contact portion between the inner surface of the side wall portion (32, 32a-b, 62, 62a-c) and the outer surface of the tubular body (21), and increases a contact area between sterilization gas and the inner surface of the side wall portion (32, 32a-b, 62, 62a-c) and the outer surface of the tubular body (21) covered by the side wall portion (32, 32a-b, 62, 62a-c).

3. The grip (30, 30a-b, 60, 60c-g) for a syringe barrel (20) according to any one of claims 1 to 2, wherein the flange receiving portion (33) includes an upper plate portion (37) and a lower plate portion (38) that sandwich the flange (22) of the barrel (20), and a slight gap through which the sterilization gas can flow is formed between the upper surface and the lower surface of the flange (22) and the upper plate portion (37) and the lower plate portion (38) of the flange receiving portion (33).

4. The grip (30, 30a-b, 60, 60c-g) for a syringe barrel (20) according to any one of claims 1 to 3, wherein a contact area between the side wall portion (32, 32a-b, 62, 62a-c) of the grip (30, 30a-b, 60, 60c-g) for a syringe barrel (20) and the proximal end portion of the tubular body (21) when the grip (30, 30a-b, 60, 60c-g) is attached to the syringe barrel (20) is 40% or less of the area of the proximal end portion of the tubular body (21) covered by the side wall portion (32, 32a-b, 62, 62a-c).

5. A barrel assembly (2, 8, 8a-c) comprising: a syringe barrel (20) including a tubular body (21) and a flange (22) provided at a proximal end of the tubular body (21) and projecting outward; and the grip (30, 30a-b, 60, 60c-g) for a syringe barrel (20) according to any one of claims 1 to 4 attached to the barrel (20).

6. A syringe (1, 7, 7a-c) comprising: the barrel assembly (2, 8, 8a-c) according to claim 5; a gasket (11) slidably received in the barrel (20); and a plunger (12) configured to move the gasket (11).

7. The syringe (1, 7, 7a-c) according to claim 6, wherein a distal end side portion of the plunger (12) is received in the barrel (20), and the plunger (12) includes a stopper portion (17) that is provided at a part to be received in the barrel (20), configured to abut on the grip (30, 30a-b, 60, 60c-g), and configured to restrict the plunger (12) from being slipped from the barrel (20).

8. The syringe (1, 7, 7a-c) according to claim 6 or 7, wherein the plunger (12) is not connected to the gasket (11) and includes a gasket pressing portion (14) that is provided at a distal end and is capable of contacting a proximal end portion of the gasket (11).

9. The syringe (1, 7, 7a-c) according to any one of claims 6 to 8, wherein a drug (50) is stored in the barrel (20).

## Patentansprüche

1. Griff (30, 30a - b, 60, 60c - g) für einen Spritzenzylinder (20), der an einem Spritzenzylinder (20) angebracht werden soll, welcher einen röhrenförmigen Körper (21) und einen an dem röhrenförmigen Körper (21) vorgesehenen, nach außen vorstehenden Flansch (22) umfasst, wobei der Griff (30, 30a - b, 60, 60c - g) umfasst:
einen Flansch-Aufnahmeabschnitt (33), der so konfiguriert ist, dass er den Flansch (22) aufnimmt; und einen Seitenwandabschnitt (32, 32a - b, 62, 62a - c), der sich in einer distalen Endrichtung von dem Flansch-Aufnahmeabschnitt (33) erstreckt und so konfiguriert ist, dass er teilweise einen proximalen Endabschnitt des röhrenförmigen Körpers (21) angrenzend an den Flansch (22) abdeckt, wobei
der Seitenwandabschnitt (32, 32a - b, 62, 62a - c) einen Passungsabschnitt (34, 34b, 64, 64a, 64c) umfasst, der so konfiguriert ist, dass er an den proximalen Endabschnitt des röhrenförmigen Körpers (21) angepasst werden kann, und einen Luftstromabschnitt (70) aufweist, der so konfiguriert ist, dass es Sterilisationsgas gestattet ist, zu einer Außenfläche des rohrförmigen Körpers (21) zu strömen, die durch den Seitenwandabschnitt (32, 32a - b, 62, 62a - c) abgedeckt ist,
wobei der Seitenwandabschnitt (32, 32a - b, 62, 62a - c) ein röhrenförmiger Abschnitt ist, der einen Befestigungsschlitz (41) für den proximalen Endabschnitt des röhrenförmigen Körpers (21) aufweist, der Befestigungsschlitz (41) eine Öffnungsbreite aufweist, die kleiner ist als ein Außendurchmesser des röhrenförmigen Körpers (21) des Zylinders (20), bei dem Prozess des Anbringens von dem Griff (30, 30a - b, 60, 60c - g) an dem Zylinder (20) wird der Befestigungsschlitz (41) durch den Zylinder (20) elastisch verformt, um sich zu erweitern, wenn der Griff (30, 30a - b, 60, 60c - g) an dem Zylinder (20) angebracht worden ist, wird der Griff (30, 30a - b, 60, 60c - g) fest an dem Zylinder (20) durch die elastische Rückstellkraft befestigt,
der Luftstromabschnitt (70) an einer Innenfläche des Seitenwandabschnitts (32, 32a - b, 62, 62a - c) vorgesehen ist, wobei der Luftstromabschnitt (70) durch einen berührungsfreien Abschnitt zwischen der Innenfläche des Seitenwandabschnitts (32, 32a - b, 62, 62a - c) und der Außenfläche des röhrenförmigen Körpers (21) ausgebildet wird, und der Luftstromabschnitt (70) durch eine Vielzahl von Aussparungen (65) gebildet wird, die an der Innenfläche des Seitenwandabschnitts (32, 32a - b, 62, 62a - c) ausgebildet sind, oder durch einen Teil zwischen einer Vielzahl von Vorsprüngen (66, 66a - b), die an der Innenfläche des Seitenwandabschnitts (32, 32a - b, 62, 62a - c) oder einem rauen Oberflächenabschnitt (68) gebildet sind, der an der Innenfläche des Seitenwandabschnitts (32, 32a - b, 62, 62a - c) ausgebildet ist.

2. Griff (30, 30a - b, 60, 60c - g) für einen Spritzenzylinder (20) nach Anspruch 1, wobei der Luftstromabschnitt (70) eine Fläche eines Kontaktabschnitts zwischen der Innenfläche des Seitenwandabschnitts (32, 32a - b, 62, 62a - c) und der Außenfläche des röhrenförmigen Körpers (21) verringert und eine Kontaktfläche zwischen Sterilisationsgas und der Innenfläche des Seitenwandabschnitts (32, 32a - b, 62, 62a - c) und der Außenfläche des von dem Seitenwandabschnitt (32, 32a - b, 62, 62a - c) bedeckten röhrenförmigen Körpers (21) vergrößert.

3. Griff (30, 30a - b, 60, 60c - g) für einen Spritzenzylinder (20) nach einem der Ansprüche 1 bis 2, wobei der Flansch-Aufnahmeabschnitt (33) einen oberen Plattenabschnitt (37) und einen unteren Plattenabschnitt (38) umfasst, die den Flansch (22) des Zylinders (20) sandwichartig umschließen, und zwischen der Oberseite und der Unterseite des Flansches (22) und dem oberen Plattenabschnitt (37) und dem unteren Plattenabschnitt (38) des Flansch-Aufnahmeabschnitts (33) ein schmaler Spalt ausgebildet ist, durch den das Sterilisationsgas strömen kann.

4. Griff (30, 30a - b, 60, 60c -g) für einen Spritzenzylinder (20) nach einem der Ansprüche 1 bis 3, wobei eine Kontaktfläche zwischen dem Seitenwandabschnitt (32, 32a - b, 62, 62a - c) des Griffs (30, 30a - b, 60, 60c - g) für einen Spritzenzylinder (20) und dem proximalen Endabschnitt des röhrenförmigen Körpers (21), wenn der Griff (30, 30a - b, 60, 60c - g) an dem Spritzenzylinder (20) befestigt ist, 40 % oder weniger der Fläche des proximalen Endabschnitts des röhrenförmigen Körpers (21) beträgt, die von dem Seitenwandabschnitt (32, 32a - b, 62, 62a - c) bedeckt ist.

5. Zylinderanordnung (2, 8, 8a - c), umfassend: einen Spritzenzylinder (20), der einen röhrenförmigen Körper (21) und einen Flansch (22) umfasst, der an einem proximalen Ende des zylindrischen Körpers angeordnet ist, und sich nach außen vorsteht; und den Griff (30, 30a - b, 60, 60c - g) für einen Spritzenzylinder (20) nach einem der Ansprüche 1 bis 4, der an dem Zylinder (20) angebracht ist.

6. Spritze (1, 7, 7a - c), umfassend: die Zylinderanordnung (2, 8, 8a - c) nach Anspruch 5; eine Dichtung (11), die verschiebbar in dem Zylinder (20) aufgenommen ist; und einen Kolben (12), der zum Bewegen der Dichtung (11) konfiguriert ist.

7. Spritze (1, 7, 7a - c) nach Anspruch 6, wobei ein distaler Endabschnitt des Kolbens (12) in dem Zylinder (20) aufgenommen ist, und der Kolben (12) einen Anschlagabschnitt (17) aufweist, der an einem in dem Zylinder (20) aufzunehmenden Teil vorgesehen ist, welcher so konfiguriert ist, dass er an dem Griff (30, 30a - b, 60, 60c - g) anliegt, und so konfiguriert ist, dass er verhindert, dass der Kolben (12) aus dem Zylinder (20) herausrutscht.

8. Spritze (1, 7, 7a - c) nach Anspruch 6 oder 7, wobei der Kolben (12) nicht mit der Dichtung (11) verbunden ist und einen Dichtungsandrückabschnitt (14) aufweist, der an einem proximalen Endabschnitt vorgesehen ist und der in der Lage ist, mit einem proximalen Endabschnitt der Dichtung (11) in Kontakt zu kommen.

9. Spritze (1, 7, 7a - c) nach einem der Ansprüche 6 bis 8, wobei ein Arzneimittel (50) in dem Zylinder (20) aufbewahrt ist.

## Revendications

1. Poignée (30, 30a-b, 60, 60c-g) de cylindre de seringue (20) destinée à être fixée à un cylindre de seringue (20) qui inclut un corps tubulaire (21), et une bride (22) prévue sur le corps tubulaire (21) et faisant saillie vers l'extérieur, dans laquelle
la poignée (30, 30a-b, 60, 60c-g) comprend :
une partie de réception de bride (33) configurée pour recevoir la bride (22) ; et une partie de paroi latérale (32, 32a-b, 62, 62a-c) s'étendant dans une direction d'extrémité distale de la partie de réception de bride (33) et configurée pour couvrir partiellement une partie d'extrémité proximale du corps tubulaire (21) adjacente à la bride (22), dans laquelle
la partie de paroi latérale (32, 32a-b, 62, 62a-c) inclut une partie d'ajustement (34, 34b, 64, 64a, 64c) configurée pour être ajustée sur la partie d'extrémité proximale du corps tubulaire (21), et une partie d'écoulement d'air (70) configurée pour permettre à du gaz de stérilisation de circuler vers une surface extérieure du corps tubulaire (21) couverte par la partie de paroi latérale (32, 32a-b, 62, 62a-c),
la partie de paroi latérale (32, 32a-b, 62, 62a-c) est une partie tubulaire ayant une fente de fixation (41) pour la partie d'extrémité proximale du corps tubulaire (21), la fente de fixation (41) présente une largeur d'ouverture plus petite qu'un diamètre extérieur du corps tubulaire (21) du cylindre (20), dans le processus de fixation de la poignée (30, 30a-b, 60, 60c-g) au cylindre (20), la fente de fixation (41) est déformée élastiquement par le cylindre (20) à élargir, lorsque la poignée (30, 30a-b, 60, 60c-g) a été fixée au cylindre (20), la poignée (30, 30a-b, 60, 60c-g) est fixée fermement au cylindre (20) par la force élastique,
la partie d'écoulement d'air (70) est prévue sur une surface intérieure de la partie de paroi latérale (32, 32a-b, 62, 62a-c), la partie d'écoulement d'air (70) est formée par une partie de non-contact entre la surface intérieure de la partie de paroi latérale (32, 32a-b, 62, 62a-c) et la surface extérieure du corps tubulaire (21), et la partie d'écoulement d'air (70) est formée par une pluralité de creux (65) formés sur la surface intérieure de la partie de paroi latérale (32, 32a-b, 62, 62a-c) ou une partie entre une pluralité de saillies (66, 66a-b) formées sur la surface intérieure de la partie de paroi latérale (32, 32a-b, 62, 62a-c) ou une partie de surface rugueuse (68) formée sur la surface intérieure de la partie de paroi latérale (32, 32a-b, 62, 62a-c).

2. Poignée (30, 30a-b, 60, 60c-g) de cylindre de seringue (20) selon la revendication 1, dans laquelle la partie d'écoulement d'air (70) réduit une aire d'une partie de contact entre la surface intérieure de la partie de paroi latérale (32, 32a-b, 62, 62a-c) et la surface extérieure du corps tubulaire (21), et augmente une aire de contact entre le gaz de stérilisation et la surface intérieure de la partie de paroi latérale (32, 32a-b, 62, 62a-c) et la surface extérieure du corps tubulaire (21) couverte par la partie de paroi latérale (32, 32a-b, 62, 62a-c).

3. Poignée (30, 30a-b, 60, 60c-g) de cylindre de seringue (20) selon l'une quelconque des revendications 1 à 2, dans laquelle la partie de réception de bride (33) inclut une partie de plaque supérieure (37) et une partie de plaque inférieure (38) qui prend en sandwich la bride (22) du cylindre (20), et un léger espace à travers lequel le gaz de stérilisation peut s'écouler est formé entre la surface supérieure et la surface inférieure de la bride (22) et la partie de plaque supérieure (37) et la partie de plaque inférieure (38) de la partie de réception de bride (33).

4. Poignée (30, 30a-b, 60, 60c-g) de cylindre de seringue (20) selon l'une quelconque des revendications 1 à 3, dans laquelle une aire de contact entre la partie de paroi latérale (32, 32a-b, 62, 62a-c) de la poignée (30, 30a-b, 60, 60c-g) de cylindre de seringue (20) et la partie d'extrémité proximale du corps tubulaire (21) lorsque la poignée (30, 30a-b, 60, 60c-g) est fixée au cylindre de seringue (20) fait 40 % ou moins de l'aire de la partie d'extrémité proximale du corps tubulaire (21) couverte par la partie de paroi latérale (32, 32a-b, 62, 62a-c).

5. Ensemble de seringue (2, 8, 8a-c) comprenant : un cylindre de seringue (20) incluant un corps tubulaire (21) et une bride (22) prévue sur une extrémité proximale du corps tubulaire (21) et faisant saillie vers l'extérieur ; et la poignée (30, 30a-b, 60, 60c-g) de cylindre de seringue (20) selon l'une quelconque des revendications 1 à 4 fixée au cylindre (20).

6. Seringue (1, 7, 7a-c) comprenant : l'ensemble de cylindre (2, 8, 8a-c) selon la revendication 5 ; un joint d'étanchéité (11) reçu coulissant dans le cylindre (20) ; et un piston (12) configuré pour déplacer le joint d'étanchéité (11).

7. Seringue (1, 7, 7a-c) selon la revendication 6, dans laquelle une partie de côté d'extrémité distale du piston (12) est reçue dans le cylindre (20), et le piston (12) inclut une partie de butée (17) qui est prévue sur une partie qui doit être reçue dans le cylindre (20), configurée pour être adjacente sur la poignée (30, 30a-b, 60, 60c-g), et configurée pour empêcher le piston (12) de glisser du cylindre (20).

8. Seringue (1, 7, 7a-c) selon la revendication 6 ou 7, dans laquelle le piston (12) n'est pas connecté au joint d'étanchéité (11) et inclut une partie de pression de joint (14) qui est prévue à une extrémité distale et est capable de contacter une partie d'extrémité proximale du joint (11).

9. Seringue (1, 7, 7a-c) selon l'une quelconque des revendications 6 à 8, dans laquelle un médicament (50) est stocké dans le cylindre (20).
